# EUROPÄISCHE PATENTSCHRIFT

(11) **EP 3 503 113 B1**
(45) Veröffentlichungstag und Bekanntmachung des Hinweises auf die Patenterteilung: **21.02.2024**
(21) Anmeldenummer: 17210280.8
(22) Anmeldetag: 22.12.2017
(51) Int. Cl.: G16H 30/20

(54) **CLOUDBASIERTE MR-BILDGEBUNG**
CLOUD-BASED MR IMAGING
IMAGERIE MR EN NUAGE

(43) Veröffentlichungstag der Anmeldung: 26.06.2019
(73) Patentinhaber: Siemens Healthineers AG, 91301 Forchheim (DE)
(72) Erfinder: Faber, Roland, 91080 Uttenreuth (DE); Blum, Thomas, 91077 Neunkirchen A. Br (DE); Wübbe, Marcus, 91074 Herzogenaurach (DE)
(74) Vertreter: Siemens Healthineers Patent Attorneys

(56) Entgegenhaltungen:
- US-A1- 2013 208 955
- US-A1- 2014 278 496

## Beschreibung

Die Erfindung betrifft ein Magnetresonanzbildgebungssystem. Weiterhin betrifft die Erfindung ein Verfahren zum Durchführen einer Magnetresonanzbildgebung.

In einem Magnetresonanzbildgebungssystem, auch Magnetresonanztomographiesystem oder Magnetresonanzanlage genannt, wird für die Erstellung von Magnetresonanzaufnahmen üblicherweise der zu untersuchende Körper mit Hilfe eines Grundfeldmagnetsystems einem relativ hohen Grundmagnetfeld, beispielsweise von 1,5 Tesla, 3 Tesla oder 7 Tesla, ausgesetzt. Zusätzlich wird mit Hilfe eines Gradientensystems ein Magnetfeldgradient angelegt. Über ein Hochfrequenz-Sendesystem werden dann mittels geeigneter Antenneneinrichtungen hochfrequente Anregungssignale (HF-Signale) ausgesendet, was dazu führen soll, dass die Kernspins bestimmter, durch dieses Hochfrequenzfeld resonant angeregter Atome um einen definierten Flipwinkel gegenüber den Magnetfeldlinien des Grundmagnetfelds verkippt werden. Bei der Relaxation der Kernspins werden Hochfrequenzsignale, so genannte Magnetresonanzsignale, abgestrahlt, die von einem Hochfrequenz-Empfangssystem mittels geeigneter Empfangsantennen empfangen und dann weiterverarbeitet werden. Aus den so akquirierten Rohdaten können schließlich die gewünschten Bilddaten rekonstruiert werden.

Die in einem herkömmlichen Magnetresonanzbildgebungssystem, kurz MR-System, genutzten Computer-Ressourcen sind nicht skalierbar und bleiben daher zum Teil oft ungenutzt oder reichen bei einer Spitzenbelastung manchmal nicht aus. Die in einem MR-System eingebetteten Computer-Ressourcen werden bisher nach Erfahrungswerten festgelegt und üblicherweise in maximal zwei unterschiedlichen Optionen angeboten. Die Ressourcen bleiben die meiste Zeit ungenutzt und verursachen unnötige Bereitstellungskosten. Liegt eine Spitzenbelastung vor, so wird der Anwender zu unproduktiven Aufräum-Aktionen gezwungen, um die benötigten Ressourcen bereitzustellen.

Soll eine neue Systemsoftware bei den Anwendern implementiert werden, so kann es oft Monate dauern, bis die Software bei den Anwendern einsatzbereit ist. Neue MR-Systemsoftware wird mittels eines Installationsmediums an die Anwender übermittelt, die eine Installation durch einen Service-Techniker vornehmen lassen. Oft ist der Vorgang mit mehrmonatigen Verzögerungen verbunden. Teilweise dauert es Jahre, bis eine neue Software vollständig im Markt "ausgerollt" bzw. eingeführt ist.

Oft müssen die Anwender, um eine neue MR-Systemsoftware einzusetzen, alle Abläufe auf eine neue Funktionalität der Software einstellen. Dabei gibt es meist keine Möglichkeit, ausreichende und gut beherrschte Abläufe beizubehalten. Weist die neue Software noch Unzulänglichkeiten auf, so kann der Anwender die alte, bewährte Software nur durch eine Uminstallation auf dem MR-System reaktivieren. Für den Anwender speziell abgestimmte Lösungen müssen aufwändig auf die neue Software migriert werden oder gehen verloren.

Großorganisationen benötigen zentral standardisierte Abläufe, die verteiltes Arbeiten flexibel unterstützen, was bei der Magnetresonanzbildgebung bisher nicht ausreichend berücksichtigt ist. Zur Standardisierung werden Arbeitsabläufe mit gro-βem Aufwand bei Anwendern mit vielen Systemen manuell kopiert. Dabei muss der leitende Technologe beispielsweise einen USB-Stick an jedes MR-System bringen lassen und dort die Daten einspielen. Wegen des hohen Zeitbedarfs sind Anpassungen des Standards sehr aufwändig.

In einem sehr allgemeinen Zusammenhang offenbaren die Schriften US20160239615A1, US20160300016A1, US9524582B2, US20170293736A1, US20170206339A1 dein Einsatz von Cloud-Technologien insbesondere im Zusammenhang mit medizinischen Bildgebungsgeräten.

Verteiltes Arbeiten wird beispielsweise durch die Funktion Expert-i unterstützt, die ein Remote Control, also eine Art Fernsteuerung eines MR-Systems in Form einer Punkt-zu-Punkt-Verbindung unterstützt. Dabei muss der Remote-Client mit dem MR-System kompatibel sein, was entweder inakzeptablen manuellen Aufwand oder kundenspezifische Speziallösungen erfordert. Ein Umplanen von Patienten auf andere MR-Systeme wird nicht unterstützt und eine Weitergabe des Workflows, d.h. des patientenspezifischen Arbeitsablaufs, an andere Arbeitsplätze ist nur mit der Einschränkung möglich, dass die Benutzeroberfläche des jeweiligen Bildgebungssystems gesperrt bleibt.

In US 2014/ 0 278 496 A1 wird ein System zur Planung und Verteilung von medizinischen Ressourcen beschrieben. Von dem System werden in einem zentralen Rechner vorhandene Ressourcen und Prozesses unterschiedlichen Untersuchungsräumen und Untersuchungsverfahren zugewiesen.

In US 2013/ 0 208 955 A1 wird ein cloudbasierte Auswertung von medizinischen Bilddaten beschrieben. Ein Cloud-Server stellt Bildbearbeitungsdienste für unterschiedliche Benutzer zur Verfügung.

Es besteht also ein Problem hinsichtlich einer vereinfachten Wartung und Anpassung von MR-Systemen in komplexen Organisationsstrukturen.

Diese Aufgabe wird durch ein Magnetresonanzbildgebungssystem gemäß Patentanspruch 1 und ein Verfahren zum Durchführen einer Magnetresonanzbildgebung gemäß Patentanspruch 5 gelöst.

Das erfindungsgemäße Magnetresonanzbildgebungssystem weist eine Infrastruktur innerhalb einer Cloud auf. Die Infrastruktur erzeugt und/oder aktiviert und/oder deaktiviert und/oder beendet virtuelle Systeme nach Bedarf. Die Infrastruktur umfasst Ressourcen, vorzugsweise eine Software bzw. ein Computerprogramm, mit denen die virtuellen Systeme erzeugt werden.

Die virtuellen Systeme erzeugen und betreiben eine Mehrzahl von Arbeitsabläufen als Instanzen. Anders ausgedrückt, werden die Arbeitsabläufe von den virtuellen Systemen über ein Kommunikationsnetz, beispielsweise das Internet, in einem Hosting-Vorgang zur Verfügung gestellt und durchgeführt. Es können mehrere Arbeitsabläufe von jeweils ein- und demselben Satz von virtuellen Systemen betrieben werden.

Jeder Arbeitsablauf enthält Informationen über die spezifische diagnostische Fragestellung für den jeweils betroffenen Patienten, etwa in Form von spezifischen Ablaufdetails und Parameterwerten für die Messung, sowie patientenspezifische Informationen wie beispielsweise Größe und Gewicht oder Informationen zur Kontrastmittel-Verträglichkeit.

Patientendaten, wie zum Beispiel Informationen über Erkrankungen, Allergien usw. beeinflussen die Arbeitsabläufe. Beispielsweise wird ein Patient mit Kontrastmittel-Allergie einer anderen Messmethode unterzogen als ein Patient, der Kontrastmittel-verträglich ist. Die Auswahl der Arbeitsabläufe richtet sich primär nach der diagnostischen Fragestellung. Sekundär wird aus der Untermenge der zur Fragestellung passenden Abläufe nach Patienten-Eigenschaften der richtige Ablauf ausgewählt und schließlich patientenspezifisch parametriert, etwa um die genaue Lage eines Organs zu berücksichtigen oder die spezifische Absorptionsrate nach Gewicht des Patienten zu berücksichtigen.

Vorzugsweise ermöglicht die Infrastruktur ein Erzeugen und ein Aktivieren und ein Deaktivieren sowie ein Beenden der virtuellen Systeme. Vorteilhaft werden virtuelle Systeme nach Bedarf erzeugt und können je nach Anwendung modifiziert oder ersetzt oder als aktive Systeme gelöscht werden, so dass Hardware-Ressourcen geschont werden und die Datenlast des Magnetresonanzbildgebungssystems reduziert werden kann. Die virtuellen Systeme umfassen mindestens einen Cloud-basierten virtuellen MR-Host mit einem aktiven MR-Arbeitsablauf, der nach Bedarf mit einer MR-Steuerungseinheit verbunden wird.

Als virtueller MR-Host soll ein virtueller Hostrechner, welcher einen Arbeitsablauf eines MR-Bildgebungsprozesses, auch als Workflow bezeichnet, speichert und bearbeitet sowie für den Betrieb des MR-Systems durchführt und zur Verfügung stellt, verstanden werden. Als Cloud-basierter virtueller MR-Host soll ein MR-Host verstanden werden, dessen Funktion, beispielsweise mit Hilfe eines in der Cloud installierten Computerprogramms, über ein Rechnernetz, beispielsweise das Internet, zur Verfügung gestellt wird, ohne dass dafür ein lokaler Hostrechner bereitgestellt werden muss und für das Erzeugen und Aktivieren von MR-Arbeitsabläufen auf lokalen Rechnern ein entsprechendes Computerprogramm installiert werden muss.

Weiterhin weist das Magnetresonanzbildgebungssystem eine MR-Scaneinheit mit einer MR-Steuerungseinheit auf. Die MR-Steuerungseinheit ist dazu eingerichtet, auf Basis eines der in dem virtuellen MR-Host abgespeicherten Arbeitsabläufe einen Bildgebungsprozess der MR-Scaneinheit zu steuern. Da Teile der Steuerung des Magnetresonanzbildgebungssystems virtualisiert sind, kann das erfindungsgemäße Magnetresonanzbildgebungssystem auch eine Mehrzahl von MR-Scaneinheiten aufweisen, die allesamt über den virtuellen MR-Host angesteuert werden.

Überdies umfasst das Magnetresonanzbildgebungssystem mindestens eine Client-Einheit zum Übermitteln von patientenspezifischen Daten an den virtuellen MR-Host und zum Auswerten von von dem virtuellen MR-Host empfangenen Bilddaten.

Vorteilhaft können die in der Cloud vorhandenen Computer-Ressourcen nach Bedarf durch einen aktivierten MR-Arbeitsablauf genutzt werden. Anstatt für jedes Einzelsystem einen Hostrechner anzuschaffen, können diese Ressourcen in die Cloud verlegt werden und für die Benutzung sogenannte Payper-Use-Verträge mit lokalen Cloud-Anbietern geschlossen werden. Die Redundanz von Computer-Ressourcen lokaler Lösungen wird vermieden. Der Anwender muss nur für diejenigen Ressourcen bezahlen, die er tatsächlich auch nutzt. Manuelle unproduktive Aufräumarbeiten zur Freistellung benötigter Ressourcen zu ungünstigen Zeitpunkten, beispielsweise, wenn ein Patient bereits auf der Liege in der MR-Scaneinheit liegt, entfallen. Vorteilhaft können auch über einen Client mit Hilfe des virtuellen MR-Hosts mehrere MR-Scaneinheiten angesprochen werden, so dass bei Bedarf eine umfassendere Bildgebung eines Patienten möglich ist, ohne dass die Eingabe von Primärdaten wiederholt werden muss.

Bei großen medizinischen Einrichtungen können Serversysteme in Form von sogenannten Private Clouds eingerichtet werden, die für Spitzenlasten die arbeitsablaufspezifischen Ressourcen flexibel skalieren.

Neue Software kann jederzeit in der Cloud bereitgestellt werden. Sobald die Software in der Cloud bereitsteht, kann der Anwender jederzeit einen MR-Arbeitsablauf mit der neuen Software starten. Mithin ist die Verbreitung von neuen Software-Versionen vereinfacht und beschleunigt. Ein langdauerndes "Ausrollen" der Software in den Markt entfällt.

Weiterhin besteht bei einer Erneuerung der Software auf dem virtuellen MR-Host kein Zwang, die alte Software zu eliminieren, denn beide Programmversionen können in der Cloud nebeneinander zur Verfügung bereitstehen. Mithin kann der Anwender entscheiden, ober er für einen Arbeitsablauf auf die neue Software-Version zugreifen möchte oder lieber die bewährte alte Programmversion mit gut beherrschten Abläufen einsetzen möchte. Sollte die neue Software noch Unzulänglichkeiten aufweisen, kann der Anwender jederzeit die alte, bewährte Software verwenden, ohne eine Uminstallation vornehmen zu müssen. Der Anwender ist auch nicht gezwungen, sein gesamtes Personal für eine neue Software zu schulen: bestimmte Spezialisten können die neue Funktionalität nutzen, während andere noch die bewährten Verfahren weiterverwenden. Anwenderspezifische Lösungen können unabhängig nach Bedarf migriert werden, da sie weiterhin dem Anwender in der älteren Variante zur Verfügung stehen.

Überdies wird eine Standardisierung eines zu nutzenden Arbeitsablaufs, insbesondere bei der Anwendung durch Großkunden, ermöglicht. Hierzu legt der Anwender einen MR-Referenz-Arbeitsablauf in der Cloud an. Sofort kann dieser Referenz-Arbeitsablauf von allen mit der Cloud verbundenen Arbeitsplätzen bzw. Clients genutzt werden. Änderungen des Referenz-Arbeitsablaufs erfolgen einmal zentral in der Cloud und sind dann sofort verfügbar.

Aktive Instanzen eines Arbeitsablaufs können von Client zu Client weitergegeben werden, wobei alle bisherigen Ergebnisse zur Weiterverarbeitung unmittelbar ohne Ladevorgang zur Verfügung stehen. Als aktive Instanzen von Arbeitsabläufen sind geladene und aktive Programme im System zu verstehen, die diese Arbeitsabläufe ausführen. Bei Unterbrechungen und zur Langzeit-Dokumentation können Arbeitsablauf-Instanzen in der Cloud abgespeichert werden. Diese gespeicherten Instanzen können nach Bedarf reaktiviert werden. Gerade Großanwender werden mittels der Cloud-Technologie optimal unterstützt. Im Zuge der Einführung der 5G-Breitbandkommunikation ist sogar eine weltweit verteilte Zusammenarbeit zur Diagnose eines Gesundheitszustands eines Patienten möglich. Darüber hinaus lassen sich die in der Cloud gespeicherten Daten zum Data-Mining nutzen.

Bei dem erfindungsgemäßen Verfahren zum Durchführen einer Magnetresonanzbildgebung werden patientenspezifische Daten von einer Client-Einheit an mindestens einen Cloud-basierten virtuellen MR-Host, welcher einen aktiven MR-Arbeitsablauf aus einer Mehrzahl von gespeicherten MR-Arbeitsabläufen in der Cloud ausführt, übermittelt, wobei der aktive MR-Arbeitsablauf für eine Messung spezifische Parameterwerte umfasst. Weiterhin wird der virtuelle MR-Host mit einer passenden MR-Scaneinheit über eine mit der MR-Scaneinheit verbundene MR-Steuerungseinheit für eine anschließende Kommunikation verbunden. Als passende MR-Scaneinheit soll eine MR-Scaneinheit bezeichnet werden, welche für den patientenspezifischen Arbeitsablauf geeignet ist. Nachfolgend wird der aktive, patientenspezifische Arbeitsablauf von dem virtuellen MR-Host an die MR-Steuerungseinheit der MR-Scaneinheit übermittelt. Auf Basis des patientenspezifischen aktiven Arbeitsablaufs wird dann die MR-Scaneinheit durch die MR-Steuerungseinheit angesteuert. Die von der MR-Scaneinheit bei dem Bildgebungsprozess erzeugten Rohdaten werden weiterhin an den virtuellen MR-Host übermittelt. Der MR-Host kann dann Funktionalitäten zur Weiterverarbeitung der Rohdaten, wie zum Beispiel zur Rekonstruktion von Bilddaten bereitstellen. Die erzeugten Bilddaten werden schließlich an mindestens eine Client-Einheit weitergegeben. Schließlich erfolgt ein Auswerten von von dem virtuellen MR-Host empfangenen Bilddaten durch die mindestens eine Client-Einheit. Das erfindungsgemäße Verfahren teilt die Vorteile des erfindungsgemäßen Magnetresonanzbildgebungssystems.

Einige Komponenten des erfindungsgemäßen Magnetresonanzbildgebungssystems können nach Ergänzung entsprechender Hardware, wie zum Beispiel geeignete Serverkapazitäten und Datenübermittlungskapazitäten in der Cloud, zum überwiegenden Teil in Form von Softwarekomponenten ausgebildet sein. Dies betrifft insbesondere Teile des Cloud-basierten MR-Hosts und der Client-Einheiten.

Eine weitgehend softwaremäßige Realisierung hat den Vorteil, dass auch schon bisher zur Datenübertragung zwischen einzelnen Komponenten von Magnetresonanzbildgebungssystemen vorhandene Rechnersysteme auf einfache Weise durch ein in der Cloud installiertes Computerprogramm dazu genutzt werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Rechnersystems einer Cloud bzw. eines Cloud-basierten Serversystems ladbar ist, mit Programmabschnitten, um alle Schritte des erfindungsgemäßen Verfahrens auszuführen, wenn das Computerprogramm in dem Magnetresonanzbildgebungssystem ausgeführt wird.

Ein solches Computerprogrammprodukt kann neben dem Computerprogramm gegebenenfalls zusätzliche Bestandteile, wie z.B. eine Dokumentation und/oder zusätzliche Komponenten, auch Hardware-Komponenten, wie z.B. Hardware-Schlüssel (Dongles etc.) zur Nutzung der Software, umfassen

Zum Transport zur Speichereinrichtung eines die Cloud unterstützenden Rechnersystems und/oder zur Speicherung an dem Rechnersystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren Beschreibungsteilen weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung des erfindungsgemäßen Magnetresonanzbildgebungssystems weisen die virtuellen Systeme eine Cloud-basierte virtuelle Bildrekonstruktionseinrichtung auf, welche dazu eingerichtet ist, Rohdaten von der MR-Scaneinheit zu empfangen und auf deren Basis Bilddaten zu rekonstruieren.

Vorteilhaft läuft die Rekonstruktion zentralisiert ab. Eine Anpassung an bestimmte Anforderungen oder ein Update müssen also nur einmal erfolgen. Die geänderte Software kann dann für die Rekonstruktion von Rohdaten von verschiedenen MR-Scaneinheiten genutzt werden.

In einer Variante des erfindungsgemäßen Magnetresonanzbildgebungssystems weisen die virtuellen Systeme einen virtuellen Host-Controller auf, welcher dazu eingerichtet ist, den Zugriff von Client-Einheiten auf den virtuellen MR-Host zu kontrollieren. Ein solcher virtueller Host-Controller kann zum Beispiel unautorisiertes Zugreifen auf den MR-Host verhindern, so dass die allgemeine Sicherheit und die Datensicherheit des MR-Systems verbessert sind.

In einer vorteilhaften Ausgestaltung des erfindungsgemäßen Magnetresonanzbildgebungssystems weist die mindestens eine Client-Einheit mindestens eine der folgenden Client-Typen auf:
- einen Scan-Verbindungs-Client,
- einen Nachbearbeitungs-Client,
- einen Wiedergabe-Client und
- einen Überweisungs-Client.

Der Scan-Verbindungs-Client erlaubt den Zugriff auf ein MR-System bzw. die Bedienung eines MR-Systems durch einen Anwender. Im einfachsten Fall dient der Scan-Verbindungs-Client als virtuelles Bedienpult der MR-Scaneinheit.

Ein Nachbearbeitungs-Client wird zur Bearbeitung der rekonstruierten Bilddaten genutzt. Beispielsweise kann diese Nachbearbeitung die Bearbeitung von Grauwerten, Filteroperationen und Faltungen sowie die Auswahl geeigneter Bilddarstellungen für die Befundung umfassen.

Der Wiedergabe-Client dient dazu, Befundungsoperationen durchzuführen. Am Bildschirm des Wiedergabe-Client werden einem Radiologen geeignet nachbearbeitete Bilder von einem Untersuchungsbereich eines Patienten bildlich dargestellt.

Der Radiologe kann anhand der bildlichen Darstellungen eine Diagnose stellen.

Ein Überweisungs-Client dient dazu, auf Basis eines Befundungsergebnisses einen Patienten in passende Facheinrichtungen zu überweisen. Vorteilhaft können die mit unterschiedlichen Aufgaben befassten Clients alle auf die zentral in der Cloud abgespeicherten Daten zugreifen, so dass keine unnötige Redundanz besteht bzw. keine mehrfach erzeugten und möglicherweise voneinander abweichenden Daten von den einzelnen Clients genutzt werden.

Die Erfindung wird im Folgenden unter Hinweis auf die beigefügten Figuren anhand von Ausführungsbeispielen noch einmal näher erläutert. Es zeigen:
FIG 1 ein Blockdiagramm, welches ein Magnetresonanzbildgebungssystem gemäß einem Ausführungsbeispiel der Erfindung veranschaulicht,
FIG 2 ein Flussdiagramm, welches ein Verfahren zum Durchführen einer Magnetresonanzbildgebung gemäß einem Ausführungsbeispiel der Erfindung veranschaulicht.

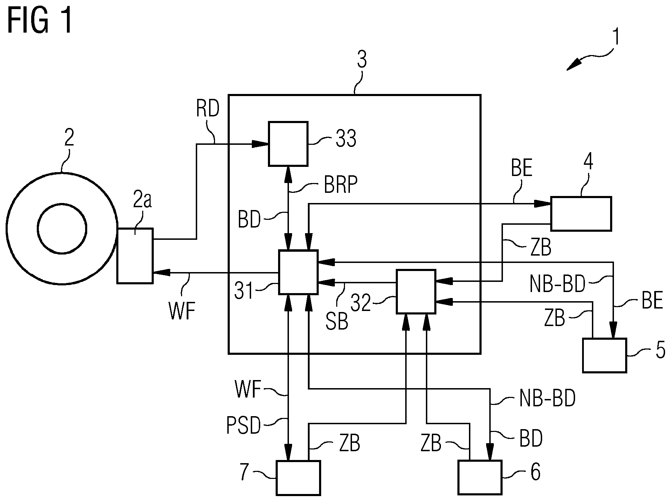

In FIG 1 ist ein Magnetresonanzbildgebungssystem 1 gemäß einem Ausführungsbeispiel der Erfindung schematisch gezeigt. Das Magnetresonanzbildgebungssystem 1 umfasst eine MR-Scaneinheit 2, die von einer MR-Steuerungseinheit 2a angesteuert wird. Die MR-Steuerungseinheit 2a erhält zum Durchführen eines Bildgebungsverfahrens an einem bestimmten Patienten einen als Arbeitsablauf WF (englisch Workflow) bezeichneten Ablaufplan, welcher auf den spezifischen Anwendungsfall abgestimmte Informationen, Messprogramme und Anweisungen aufweist. Arbeitsabläufe WF für unterschiedliche Anwendungsfälle sind in einer Cloud 3 zentral gespeichert und werden nach Bedarf in Form eines virtuellen MR-Host-Rechners 31, der je nach Bedarf mit einer MR-Scaneinheit 2 verbunden ist, aktiviert. Für die Auswahl und Konfiguration des MR-Arbeitsablaufs WF werden zum Beispiel patientenspezifische Daten PSD mit Hilfe eines Scan-Connection-Client 7 eingegeben. Zwar ist in FIG 1 nur eine Scaneinheit 2 eingezeichnet, jedoch kann der virtuelle MR-Host 31 auch mit unterschiedlichen MR-Scaneinheiten verbunden sein. Von der MR-Scaneinheit 2 werden Rohdaten RD im Rahmen eines MR-Bildgebungsprozesses eines Patienten erzeugt, die an die MR-Steuerungseinheit 2a ausgegeben werden und von dort an eine in der Cloud 3 befindliche bzw. in der Cloud 3 erzeugte virtuelle Bildrekonstruktionseinrichtung 33 übermittelt werden. Die virtuelle Bildrekonstruktionseinrichtung 33 rekonstruiert auf Basis der erhaltenen Rohdaten RD Bilddaten BD. Ein eventuell auf den spezifischen Anwendungsfall abgestimmtes Rekonstruktionsprogram BRP erhält die virtuelle Rekonstruktionseinrichtung 33 von dem virtuellen MR-Host 31. Die erzeugten Bilddaten BD werden von der virtuellen Bildrekonstruktionseinrichtung 33 an den MR-Host 31 übermittelt. Der virtuelle MR-Host 31 speichert die Bilddaten BD zentral in der Cloud 3 und übermittelt die Bilddaten BD an unterschiedliche Client-Einheiten 4, 5, 6, 7. Die einzelnen Client-Einheiten 4, 5, 6, 7 erhalten Zugriff auf den virtuellen MR-Host 31 mit Hilfe eines virtuellen Host-Controllers 32, welcher den Zugriff der Client-Einheiten 4, 5, 6, 7 auf den virtuellen MR-Host 31 regelt und überwacht und gegebenenfalls mit Steuerbefehlen SB einen Zugriff auf den virtuellen MR-Host 31 ermöglicht.

Der erwähnte Scan-Connection-Client 7, auch als Bedienpult des Technologen bezeichnet, wird zu Darstellung der Arbeitsabläufe am Bildschirm sowie für Primär-Eingaben genutzt. Primäreingaben umfassen Patienteninformation und spezifische Werte von Untersuchungs- und Bild-Berechnungsparametern. Im Rahmen einer Bildgebung erfolgt zunächst eine Messvorbereitung über den genannten Scan-Connection-Client 7, wobei ein geeigneter Arbeitsablauf WF in Form der genannten virtuellen Systeme 31, 32, 33 erzeugt und entsprechend angepasst wird. Im weiteren Verlauf des Bildgebungsvorgangs wird nun gesteuert durch die MR-Steuerungseinheit 2a und unter Anwendung des erzeugten Arbeitsablaufs WF die eigentliche Bildakquisition von einem Patienten, welche eine Akquisition von Rohdaten RD durch die Scaneinheit 2 und eine Rekonstruktion von Bilddaten BD in der virtuellen Bildrekonstruktionseinrichtung 33 umfasst, durchgeführt. Die rekonstruierten Bilddaten BD werden dann von einem Post-Processing-Client 6, der der Nachverarbeitung der Bilddaten BD dient, aus dem virtuellen MR-Host 31 abgerufen. Hierzu wird zunächst wieder eine Zugriffsanfrage ZB an den Host-Controller 32 gerichtet, der nach Prüfung der Zugriffsvoraussetzungen den Zugriff auf den virtuellen MR-Host 31 gewährt, um von dort die rekonstruierten Bilddaten BD auszulesen. Die nachbearbeiteten Bilddaten NB-BD werden nachfolgend wieder in dem virtuellen MR-Host 31 abgelegt, um für nachfolgende Auswertungsschritte zur Verfügung zu stehen. Ein sogenannter Reporting-Client 5 dient der Befundung auf Basis der gegebenenfalls nachbearbeiteten Bilddaten NB-BD. Hierzu wird wieder eine Zugriffsanfrage ZB an den Host-Controller 32 gerichtet, welcher den Zugriff auf den virtuellen MR-Host 31 gewährt, um von dort nachbearbeitete Bilddaten NB-BD auszulesen. Die nachbearbeiteten Bilddaten NB-BD werden dann auf einem Bildschirm des Reporting Client 5 bildlich dargestellt und für eine Befundung genutzt. Das Befundungsergebnis BE wird wieder an den virtuellen MR-Host 31 zurückübermittelt und dort abgespeichert. Eine eventuell notwendige Überweisung erfolgt mit Hilfe des Referring Client 4, welche sich auf die in dem MR-Host 31 abgespeicherten Befundungsergebnisse BE stützt.

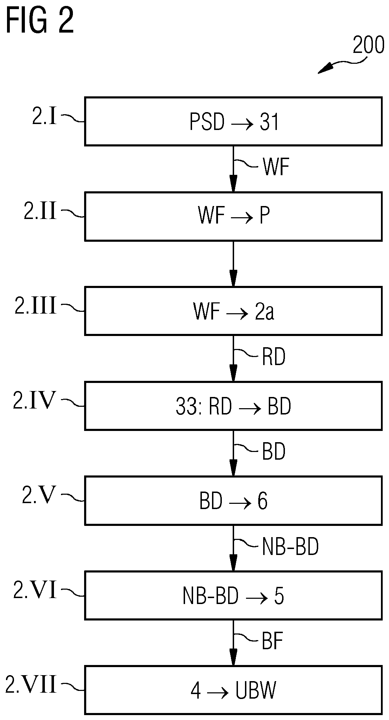

In FIG 2 ist ein Flussdiagramm 200 gezeigt, welches ein Verfahren zum Durchführen einer Magnetresonanzbildgebung veranschaulicht. Bei dem Schritt 2.1 werden patientenspezifische Daten PSD von einem Scan-Connection-Client 7 an einen Cloud-basierten virtuellen MR-Host 31, welcher eine Mehrzahl von MR-Arbeitsabläufen umfasst, übermittelt und es wird gegebenenfalls auch ein geeigneter Arbeitsablauf WF in dem virtuellen MR-Host 31 ausgewählt. Der Arbeitsablauf WF wird zum Beispiel unter Berücksichtigung von medizinischem Vorwissen über den betreffenden Patienten P, wie zum Beispiel vorhandene Bilder einer Voruntersuchung, Laborwerte, Unverträglichkeiten usw., aktiviert. Der vorbereitete Arbeitsablauf WF wird bei dem Schritt 2.II dann von einem Mess-Spezialisten für das verwendete MR-System übernommen und dem MR-System und dem Patienten P fest zugeordnet. Falls als nötig erachtet, kann der Mess-Spezialist den Arbeitsablauf WF auch an ein anderes MR-System anpassen und diesem zuweisen. Anschließend wird bei dem Schritt 2.III der patientenspezifische Arbeitsablauf WF von dem virtuellen MR-Host 31 an eine MR-Steuerungseinheit 2a einer ausgewählten MR-Scaneinheit 2 übermittelt. Bei dem Schritt 2.III wird weiterhin die MR-Scaneinheit 2 durch die MR-Steuerungseinheit 2a auf Basis des patientenspezifischen Arbeitsablaufs WF derart angesteuert, dass von dem Patienten Rohdaten RD akquiriert werden und an eine Cloud-basierte Bildrekonstruktionseinrichtung 33 übermittelt werden. Bei dem Schritt 2.IV werden von der Bildrekonstruktionseinrichtung 33 anhand der Rohdaten RD Bilddaten BD rekonstruiert. Die Bilddaten BD werden im Folgenden bei dem Schritt 3.V aus der Cloud 3 an eine Nachbearbeitungs-Einheit, auch Post-Processing-Client 6 genannt, übermittelt. Dort werden von einem speziell geschulten Technologen die für eine Diagnose optimalen Bilddarstellungen, auch Hangings genannt, vorbereitet und durch eine automatisierte Routine-Nachverarbeitung ergänzt, so dass nachbearbeitete Bilddaten NB-BD entstehen. Anschließend werden bei dem Schritt 3.VI die nachbearbeiteten Bilddaten NB-BD an einen Reporting Client 5 übermittelt und dort von einem Radiologen begutachtet, wobei dieser einen Befund BF erstellt. Bei dem Schritt 3.VII wird schließlich von einem Überweisungs-Client 4 eine Überweisung UBW entsprechend dem ermittelten Befund BF erstellt.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Magnetresonanzbildgebungssystem (1), aufweisend:
- eine Infrastruktur innerhalb einer Cloud (3), welche dazu eingerichtet ist, virtuelle Systeme (31, 32, 33), welche eine Mehrzahl von MR-Arbeitsabläufen (WF) als Instanzen er-zeugen und betreiben, nach Bedarf zu erzeugen und/oder zu aktivieren und/oder zu deaktivieren und/oder zu beenden, wobei die virtuellen Systeme (31, 32, 33) einen Cloud-basierten virtuellen MR-Host (31) mit einem aktiven MR-Arbeitsablauf (WF), der nach Bedarf mit einer MR-Steuerungseinheit (2a) verbunden wird, umfassen, wobei der aktive MR-Arbeitsablauf (WF) für eine Messung spezifische Parameterwerte umfasst,
- eine MR-Scaneinheit (2) mit einer MR-Steuerungseinheit (2a), welche dazu eingerichtet ist, auf Basis eines der in dem virtuellen MR-Host (31) abgespeicherten Arbeitsabläufen (WF), welcher für eine Messung spezifische Parameterwerte umfasst, einen Bildgebungsprozess der MR-Scaneinheit (2) zu steuern, und
- mindestens eine Client-Einheit (4, 5, 6, 7) zum Übermitteln von patientenspezifischen Daten (PSD) an den virtuellen MR-Host (31) und zum Auswerten von von dem virtuellen MR-Host (31) empfangenen Bilddaten (BD),
**dadurch gekennzeichnet, dass**
der virtuelle MR-Host (31) ausgelegt ist, zum Durchführen des Bildgebungsprozesses an einem bestimmten Patienten an eine MR-Steuerungseinheit (2a) einer ausgewählten MR-Scaneinheit (2) den Arbeitsablauf (WF) zu übermitteln, welcher auf den spezifischen Anwendungsfall abgestimmte Informationen, Messprogramme und Anweisungen aufweist.

2. Magnetresonanzbildgebungssystem nach Anspruch 1, wobei die virtuellen Systeme eine Cloud-basierte virtuelle Bildrekonstruktionseinrichtung (33), welche dazu eingerichtet ist, Rohdaten (RD) von der MR-Scaneinheit (2) zu empfangen und auf deren Basis Bilddaten (BD) zu rekonstruieren, aufweisen.

3. Magnetresonanzbildgebungssystem nach Anspruch 1 oder 2, wobei die virtuellen Systeme einen virtuellen Host-Controller (32) aufweisen, welcher dazu eingerichtet ist, den Zugriff von Client-Einheiten (4, 5, 6, 7) auf den virtuellen MR-Host (31) zu kontrollieren.

4. Magnetresonanzbildgebungssystem nach einem der vorstehenden Ansprüche, wobei die mindestens eine Client-Einheit (4, 5, 6, 7) mindestens einen der folgenden Client-Typen umfasst:
- einen Scan-Verbindungs-Client (7),
- einen Nachbearbeitungs-Client (6),
- einen Wiedergabe-Client (5) und
- einen Überweisungs-Client (4).

5. Verfahren zum Durchführen einer Magnetresonanzbildgebung, aufweisend die Schritte:
- Übermitteln von patientenspezifischen Daten (PSD) von einer Client-Einheit (7) an einen Cloud-basierten virtuellen MR-Host (31), welcher einen aktiven MR-Arbeitsablauf (WF) aus einer Mehrzahl von MR-Arbeitsabläufen (WF) in der Cloud (3) ausführt, wobei der aktive MR-Arbeitsablauf (WF) für eine Messung spezifische Parameterwerte umfasst,
- Verbinden des virtuellen MR-Hosts (31) mit einer passenden MR-Scaneinheit (2) über eine mit der MR-Scaneinheit (2) verbundene MR-Steuerungseinheit (2a),
- Übermitteln des aktiven Arbeitsablaufs (WF) von dem virtuellen MR-Host (31) an die MR-Steuerungseinheit (2a) der MR-Scaneinheit (2),
- Ansteuern der MR-Scaneinheit (2) durch die MR-Steuerungseinheit (2a) auf Basis des aktiven Arbeitsablaufs (WF),
- Auswerten von von dem MR-Host (31) empfangenen Bilddaten (BD) durch mindestens eine Client-Einheit (6),
wobei der Arbeitsablauf (WF) auf den spezifischen Anwendungsfall abgestimmte Informationen, Messprogramme und Anweisungen aufweist.

6. Computerprogrammprodukt mit einem Computerprogramm, welches direkt in eine Speichereinheit einer Rechnereinheit eines Cloud-basierten Serversystems (3) ladbar ist, mit Programmabschnitten, um alle Schritte des Verfahrens nach Anspruch 5 auszuführen, wenn das Computerprogramm in der Rechnereinheit ausgeführt wird.

7. Computerlesbares Medium, auf welchem von einer Rechnereinheit ausführbare Programmabschnitte gespeichert sind, um alle Schritte des Verfahrens nach Anspruch 5 auszuführen, wenn die Programmabschnitte von der Rechnereinheit ausgeführt werden.

## Claims

1. Magnetic resonance imaging system (1), having:
- an infrastructure within a cloud (3), which is designed to generate and/or activate and/or deactivate and/or terminate virtual systems (31, 32, 33) on demand, which generate and operate a plurality of MR workflows (WF) as instances, wherein the virtual systems (31, 32, 33) comprise a cloud-based virtual MR host (31) with an active MR workflow (WF), which is connected on demand to an MR control unit (2a), wherein the active MR workflow (WF) comprises parameter values specific to a measurement,
- an MR scan unit (2) with an MR control unit (2a), which is designed to control an imaging process of the MR scan unit (2) on the basis of one of the workflows (WF) stored in the virtual MR host (31), said workflow comprising parameter values specific to a measurement, and
- at least one client unit (4, 5, 6, 7) for transmitting patient-specific data (PSD) to the virtual MR host (31) and for evaluating image data (BD) received by the virtual MR host (31),
**characterised in that**
the virtual MR host (31) is designed, for the purpose of carrying out the imaging process on a specific patient, to transmit the workflow (WF) to an MR control unit (2a) of a selected MR scan unit (2), which workflow has information, measuring programs and instructions tailored to the specific use case.

2. Magnetic resonance imaging system according to claim 1, wherein the virtual systems have a cloud-based virtual image reconstruction device (33), which is designed to receive raw data (RD) from the MR scan unit (2) and to reconstruct image data (BD) on the basis of that raw data.

3. Magnetic resonance imaging system according to claim 1 or 2, wherein the virtual systems have a virtual host controller (32), which is designed to control the access of client units (4, 5, 6, 7) to the virtual MR host (31).

4. Magnetic resonance imaging system according to one of the preceding claims, wherein the at least one client unit (4, 5, 6, 7) comprises at least one of the following client types:
- a scan connection client (7),
- a postprocessing client (6),
- a rendering client (5) and
- a referring client (4).

5. Method for carrying out a magnetic resonance imaging procedure, having the steps:
- transmitting patient-specific data (PSD) from a client unit (7) to a cloud-based virtual MR host (31), which executes one active MR workflow (WF) from a plurality of MR workflows (WF) in the cloud (3), wherein the active MR workflow (WF) comprises parameter values specific to a measurement,
- connecting the virtual MR host (31) to a suitable MR scan unit (2) by way of an MR control unit (2a) connected to the MR scan unit (2),
- transmitting the active workflow (WF) from the virtual MR host (31) to the MR control unit (2a) of the MR scan unit (2),
- activating the MR scan unit (2) by means of the MR control unit (2a) on the basis of the active workflow (WF),
- evaluating image data (BD) received from the MR host (31) by means of at least one client unit (6),
wherein the workflow (WF) has information, measuring programs and instructions tailored to the specific use case.

6. Computer program product with a computer program which can be loaded directly into a storage unit of a computing unit of a cloud-based server system (3), having program portions in order to carry out all the steps of the method according to claim 5 when the computer program is executed in the computing unit.

7. Computer-readable medium on which program portions which can be executed by a computer are stored in order to carry out all the steps of the method according to claim 5 when the program portions are executed by the computing unit.

## Revendications

1. Système (1) d'imagerie par résonnance magnétique, comportant :
- une infrastructure au sein d'un nuage (3), qui est agencée pour, suivant les besoins, produire et/ou activer et/ou désactiver et/ou terminer des systèmes (31, 32, 33) virtuels, qui produisent et font fonctionner, comme instances, une pluralité de déroulements (WF) de travail RM, dans lequel les systèmes (31, 32, 33) virtuels comprennent un hôte (31) RM virtuel reposant sur un nuage et ayant un déroulement (WF) de travail RM actif, qui est relié suivant les besoins à une unité (2a) de commande RM, dans lequel le déroulement (WF) de travail RM actif comprend, pour une mesure de valeurs de paramètres spécifiques,
- une unité (2) de scan RM ayant une unité (2a) de commande RM, qui est agencée pour, sur la base de l'un des déroulements (WF) de travail mis en mémoire dans l'hôte (31) RM virtuel et comprenant des valeurs de paramètres spécifiques à une mesure, commander un processus d'imagerie de l'unité (2) de scan RM, et
- au moins une unité (4, 5, 6, 7) de client pour la transmission de données (PSD) spécifiques à un patient à l'hôte (31) RM virtuel et pour l'exploitation de données (BD) d'image reçues par l'hôte (31) RM virtuel,
**caractérisé en ce que**
l'hôte (31) RM virtuel est conçu, pour effectuer le processus d'imagerie sur un patient déterminé, pour transmettre le déroulement (WF) de travail à une unité (2a) de commande RM d'une unité (2) de scan RM sélectionnée, lequel déroulement a des informations, des programmes de mesure et des instructions adaptés au cas d'application spécifique.

2. Système d'imagerie par résonnance magnétique suivant la revendication 1, dans lequel les systèmes virtuels ont un dispositif (33) de reconstruction d'image virtuelle reposant sur un nuage, qui est agencé pour recevoir des données (RD) brutes de l'unité (2) de scan RM et pour reconstruire des données (BD) d'image sur leurs bases.

3. Système d'imagerie par résonnance magnétique suivant la revendication 1 ou 2, dans lequel les systèmes virtuels ont une unité (32) de commande d'hôte virtuel, qui est agencée pour contrôler l'accès d'unités (4, 5, 6, 7) de client à l'hôte (31) RM virtuel.

4. Système d'imagerie par résonnance magnétique suivant l'une des revendications précédentes, dans lequel la au moins une unité (4, 5, 6, 7) de client comprend au moins l'un des types de clients suivants :
- un client (7) de liaison de scan,
- un client (6) de post-traitement,
- un client (5) de restitution et
- un client (4) d'annotation.

5. Procédé pour effectuer une imagerie par résonnance magnétique, comprenant les stades :
- transmission de données (PSD) spécifiques à un patient d'une unité (7) de client à un hôte (31) RM virtuel reposant sur un nuage, qui exécute dans le nuage (3), un déroulement (WF) de travail RM actif parmi une pluralité de déroulements (WF) de travail RM, dans lequel le déroulement (WF) de travail RM actif comprend, pour une mesure, des valeurs de paramètres spécifiques,
- liaison de l'hôte (31) RM virtuel à une unité (2) de scan RM adaptée par une unité (2a) de commande RM reliée à l'unité (2) de scan RM,
- transmission du déroulement (WF) de travail actif de l'hôte (31) RM virtuel à l'unité (2a) de commande RM de l'unité (2) de scan RM,
- commande de l'unité (2) de scan RM par l'unité (2a) de commande RM sur la base du déroulement (WF) de travail actif,
- exploitation, par au moins une unité (6) de client, de données (BD) d'image reçues par l'hôte (31) RM,
dans lequel
le déroulement (WF) de travail a des informations, des programmes de mesure et des instructions adaptés au cas d'application spécifique.

6. Produit de programme d'ordinateur comprenant un programme d'ordinateur, qui peut être chargé directement dans une unité de mémoire d'une unité informatique d'un système (3) serveur reposant sur un nuage, comprenant des parties de programme pour effectuer tous les stades du procédé suivant la revendication 5, lorsque le programme d'ordinateur est réalisé dans l'unité informatique.

7. Support, déchiffrable par ordinateur, sur lequel sont mis en mémoire des parties de programme pouvant être réalisées par une unité informatique, afin d'effectuer tous les stades du procédé suivant la revendication 5, lorsque les parties de programme sont réalisées par l'unité informatique.
